# EUROPÄISCHE PATENTANMELDUNG

(11) **EP 3 158 980 A1**
(43) Veröffentlichungstag der Anmeldung: **26.04.2017**
(21) Anmeldenummer: 15003005.4
(22) Anmeldetag: 20.10.2015
(51) Int. Cl.: A61F 13/42

(54) **GANDY WINDELSENSOR**

(71) Anmelder: Gady, Shapira, 74074 Heilbronn (DE); Friedrich, Andreas, 71696 Möglingen (DE)
(72) Erfinder: Shapira, Gady, 74223 Flein (DE); Andreas, Friedrich, 71696 Möglingen (DE)

(57) **Zusammenfassung**

Gandy ist ein System zur Überwachung einer Windel bzw. des Trägers. Gandy ist ein Sensor der entweder in einer Windel fest integriert ist oder auch als Einlage verwendet werden kann. An diesem Sensor ist eine Elektronik angebracht, die via Bluetooth Smart (4.0) mehrere Werte auf ein Smartphone, Tablet, PC oder anderes Bluetooth-fähiges Gerät übertragen kann. Ist eine Verunreinigung einer Windel geschehen, kann dann die Elektronik einfach entfernt werden und an die neue Windel bzw. Einlage geknipst werden. Somit wird nur die Windel incl. Sensor erneuert, nicht aber die Elektronik. Die Elektronik wird über eine handelsübliche Knopfzelle CR2032 gespeist und hat eine Laufzeit (im 24/7-Betrieb) von ca. einem Jahr.

## Beschreibung

### Technisches Gebiet

Die Erfindung betrifft ein Verfahren zur Messung des Feuchtigkeitsgehalts in einer Windel die während der Messung am Körper getragen wird. Die Messwerte werden dabei via Bluetooth 4.0 zu einem Empfänger, vorzugsweise einem Smartphone, übertragen um dort weiterverarbeitet zu werden.

Nach einer Verunreinigung der Windel wird der Sensor vom Bluetooth-Sender getrennt, die Windel inklusive dem verunreinigten Sensor entsorgt und der Bluetooth-Sender an einen neuen Sensor angeknipst und wieder in eine neue Windel gelegt. Aus Hygienegesichtspunkten wird der Sensor also immer erneuert, der Bluetooth-Sender bleibt immer bestehen.

### Stand der Technik

Aus dem Stand der Technik sind Messverfahren bekannt, die kabelgebunden sind oder Verfahren die mittels Tinte einen entsprechenden Zustand einer Windel außen anzeigen können.

Der Einsatz der kabelgebundenen Lösung schränkt einen Träger erheblich in seiner Bewegungsfreiheit ein und kann ggf. beschädigt werden, wenn ein Träger die maximale Kabellänge gewollt oder ungewollt überschreitet.

Das Verfahren mit der Tinte hat den entscheidenden Nachteil, dass man einen Träger ausziehen muss um den Zustand der Windel zu prüfen. Zudem kann eine Verunreinigung längere Zeit unbemerkt bleiben.

### Darstellung der Erfindung

Der vorliegenden Erfindung liegt die Aufgabe zu Grunde, eine zeitnahe Übermittlung des Feuchtigkeitsgehalts einer Windel, drahtlos an einen Empfänger z.B. eine Aufsichtsperson oder in ein Schwesternzimmer, zu übermitteln. Dadurch kann zeitnah und nur wenn notwendig, eine Windel gewechselt werden. Dies hat Vorteile für den Träger, der nicht mehr als notwendig mit einer verunreinigten Windel ausharren muss und Vorteile für den Empfänger, der nun nicht mehr selbst aktiv werden muss, sondern informiert wird, wenn eine Windel verunreinigt wurde. Zudem lassen sich (nach Zustimmung des Trägers) auch elektronische Auswertungen darüber erstellen, wie oft und zu welchen Tageszeiten Verunreinigungen vorkommen. Dadurch wird außerdem die Planung für die Nachbestellung von Windel besser unterstützt.

### Kurzbeschreibung der Zeichnungen

Weitere Ziele, Merkmale, Vorteile und Anwendungsmöglichkeiten der erfindungsgemäßen Vorrichtung ergeben sich aus der nachfolgenden Beschreibung von Ausführungsbeispielen anhand der Zeichnungen. Dabei bilden alle beschriebenen und/oder bildlichen dargestellten Merkmale für sich oder in beliebiger Kombination den Gegenstand der Erfindung, unabhängig von der Zusammenfassung in einzelnen Ansprüchen oder deren Rückbeziehung.

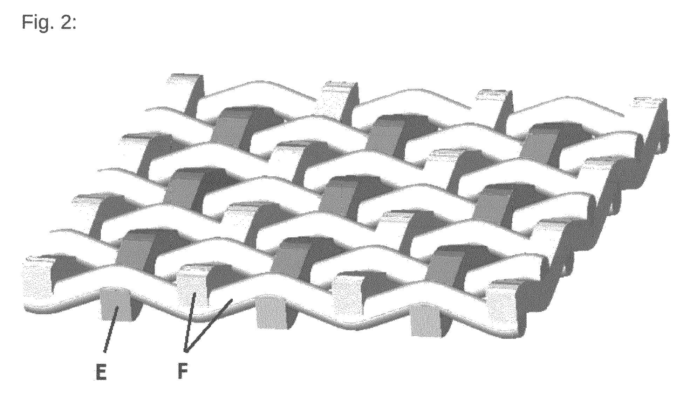

In den Zeichnungen zeigen:

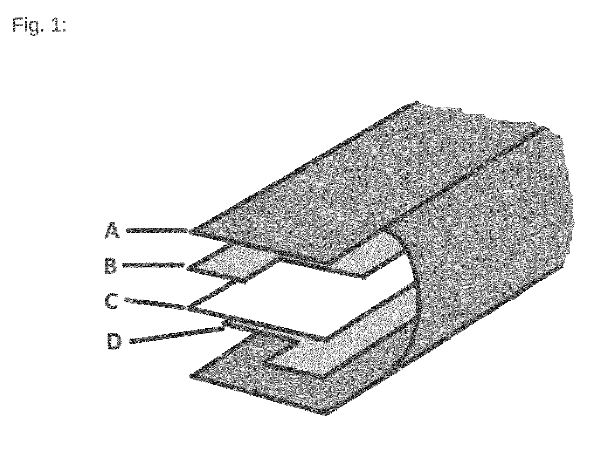

Fig. 1 der Aufbau eines Sensors für die Bestimmung des Feuchtigkeitsgehalts.
   Der Sensor besteht außen aus einem saugfähigen Flies (A). Die Lagen (B) und (D) sind zwei Sensorteile, wie unter Fig. 2 beschrieben und durch einen Isolator(C), einem saugfähigem Flies, getrennt. Die lagen (B) und (D) weisen am Ende für den Kontakt zur Elektronik zwei Laschen auf. Diese können durch alle Lagen hindurch mit einem Druckknopf in elektrische Verbindung gebracht werden.
Fig. 2 zeigt eine Lage z.B. (B) aus Fig. 1 im Detail.
   Die einzelne Lage ist aus einem elektrisch nicht leitendem Gewebe aufgebaut (F). Dieses wird durchzogen von mehreren einzelnen sehr dünnen und flexiblen Adern eines leitenden Metalls (E), z.B. Edelstahl. Zudem sind in regelmäßigen Abständen Verbindungs-Adern im 90° Winkel eingearbeitet um die längs verlaufenden Adern zu verbinden.

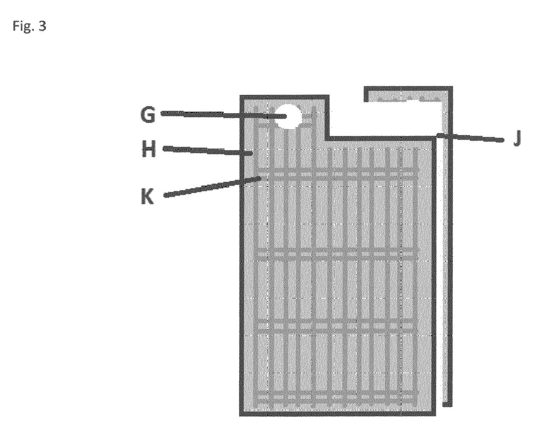

Fig. 3 zeigt eine Lage (H) in der Draufsicht.
   Die Markierung (K) zeige die Stelle an der ein Druckknopf eingepresst wird. (K) stellt die leitfähigen Adern schematisch dar. (J) ist der Isolator. Die zweite Lage ist genauso aufgebaut, allerdings spiegelverkehrt auf dem Isolator angebracht.
   das Ende des Sensors an dem der Bluetooth-Sender angebracht wird.

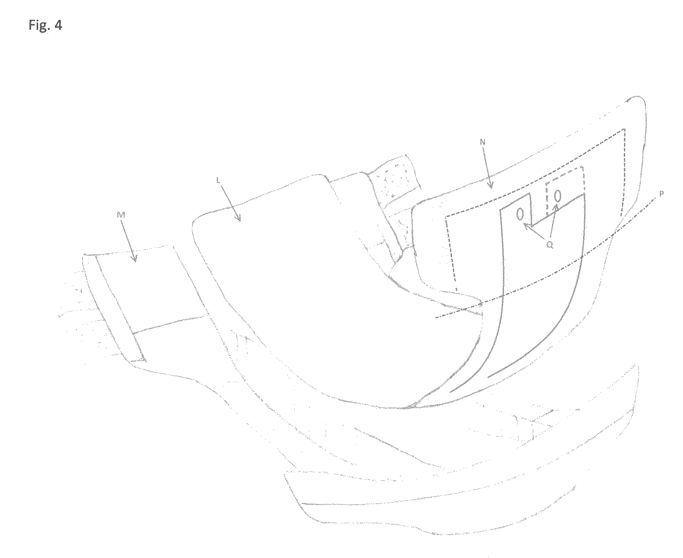

Fig. 4 die Befestigung des Sensors (L) an einer Windel (M).
   Die Elektronik wird an den zwei Druckknöpfen (Q) angeknipst. Dann wird der Sensor an der Kante (P) nach unten geknickt. Die Kante (P) wird dabei auf der oberen forderen Windel-Kante aufgelegt. Mit den Klebestellen (N) wird der Sensor dann auf die Vorderseite der Windel geklebt und schließt somit gleichzeitig die Elektronik ein. Der übrige Sensor wird einfach auf die innere Sitzfläche der Windel gelegt.

### Ausführung der Erfindung

Die Elektronik misst mittels eines Analog/Digital-Wandlers und einem in Reihe geschaltenen 3MOhm Widerstandes bei 3V. Daraus ergibt sich ein Maximal-Strom von 0,000001 Ampere (1 µA) im Falle einer Verunreinigung z.B. durch Urin (entspricht fast einem Kurzschluss). Da ein Stromkreis nur zwischen den Bahnen 2 + 4 existieren kann, besteht zu keiner Zeit eine Gefahr für den Träger.

### Anwendungsbereiche

Mögliche Anwendungsbereiche sind:
- Windeln für Babys und Kleinkinder
- Windeln für Erwachsene z.B. in Altenpflege oder betreutes Wohnen
- Krankenhaus
- Private Pflegedienste

In einer späteren Version kann man z.B. in einem Krankenhaus eine ,Meldezentrale'-App haben, auf der die Zimmer und Patienten der Station abgebildet sind. Tritt eine Verunreinigung ein, so wird der entsprechende Patient auf dem Display markiert und das Personal informiert. Eine Historie der Verunreinigungen kann ebenfalls gespeichert und ausgewertet werden.

### Vorteile

Hier einige Vorteile, die Gandy mit sich bring:
- Das Baby, der Erwachsene oder der Patient liegen nicht mehr unbemerkt und unnötig lange in einer vollen Windel.
- Es muss keine Sicht- bzw. Geruchs-Kontrolle gemacht werden.
- Windeln werden nicht unnötig oft gewechselt.
- Die Elektronik ist wiederverwendbar.
- Die Batterie wird ca. 1 Jahr im 24/7-Betrieb halten.
- Einfacher und kostengünstiger, Einlege'-Sensor bzw. der Aufpreis zu einer herkömmlichen Windel ist gering.

## Patentansprüche

1. Geschützt werden, soll das Verfahren zur Feuchtigkeitsmessung in einem Hygieneartikel mittels eines Analog-/Digital-Wandlers.

2. Geschützt werden, soll der Aufbau des Sensors zur Feuchtigkeitsmessung. Bestehend aus 2 Lagen mit nicht leitendem Gewebe, das einzelne Adern aus sehr dünnen und flexiblen leitendem Metall besitzt, die durch ein saugfähigen Isolator getrennt sind (z.B. einem Flies).

3. Geschützt werden soll der Name "Gandy" als Name für das Produkt mit dem eine Feuchtigkeitsmessung in einem Hygieneartikel gemacht werden kann.
